# EUROPÄISCHE PATENTSCHRIFT

(11) **EP 1 107 942 B1**
(45) Veröffentlichungstag und Bekanntmachung des Hinweises auf die Patenterteilung: **12.05.2004**
(21) Anmeldenummer: 99944417.7
(22) Anmeldetag: 17.08.1999
(51) Int. Cl.: C07C 209/48, C07C 211/12

(54) **VERBESSERTES VERFAHREN ZUR HERSTELLUNG VON HEXAMETHYLENDIAMIN**
IMPROVED METHOD FOR PRODUCING HEXAMETHYLENE DIAMINE
AMELIORATION APPORTEE A UN PROCEDE DE PREPARATION D'HEXAMETHYLENE-DIAMINE

(30) Priorität: 28.08.1998 DE 19839346
(43) Veröffentlichungstag der Anmeldung: 20.06.2001
(73) Patentinhaber: BASF AKTIENGESELLSCHAFT, 67056 Ludwigshafen (DE)
(72) Erfinder: FISCHER, Rolf, D-69121 Heidelberg (DE); BASSLER, Peter, D-68519 Viernheim (DE); LUYKEN, Hermann, D-67069 Ludwigshafen (DE); OHLBACH, Frank, D-69221 Dossenheim (DE); MELDER, Johann-Peter, D-67459 Böhl-Iggelheim (DE); MERGER, Martin, D-67227 Frankenthal (DE); ANSMANN, Andreas, D-69168 Wiesloch (DE); REHFINGER, Alwin, D-67112 Mutterstadt (DE); VOIT, Guido, D-67251 Freinsheim (DE)
(86) Internationale Anmeldenummer: PCT/EP1999/006014
(87) Internationale Veröffentlichungsnummer: WO 2000/012460

(56) Entgegenhaltungen:
- WO-A-98/11060
- US-A- 3 696 153
- US-A- 4 064 172

## Beschreibung

Die Anmeldung betrifft ein Verfahren zur katalytischen Hydrierung von Adipodinitril zu Hexamethylendiamin bei erhöhter Temperatur und erhöhtem Druck in Gegenwart von Katalysatoren, die auf elementarem Eisen als katalytisch aktive Komponente basieren, und Ammoniak als Lösungsmittel, dadurch gekennzeichnet, daß man
a) Adipodinitril bei Temperaturen von 70 bis 220 C und Drücken von 100 bis 400 bar in Gegenwart von Katalysatoren, die auf elementarem Eisen als katalytisch aktive Komponente basieren, und Ammoniak als Lösungsmittel zu einer Mischung enthaltend Adipodinitril, 6-Amincapronitril, Hexamethylendiamin und Hochsieder hydriert, bis die Summe aus der 6-Aminocapronitril- und der Adipodinitril-Konzentration von 1 bis 50 Gew.-%, bezogen auf das ammoniakfreie Hydriergemisch, beträgt,
b) aus dem Hydrieraustrag Ammoniak abtrennt,
c) aus dem verbleibenden Gemisch Hexamethylendiamin abtrennt,
d) 6-Aminocapronitril und Adipodinitril von Hochsiedern einzeln oder gemeinsam abtrennt und
e) 6-Aminocapronitril, Adipodinitril oder deren Gemische in den Schritt a) zurückführt.

Es ist aus US 3.696.153 bekannt, Adipodinitril bei Temperaturen von 100 bis 200°C und Drücken von etwa 340 atm in Gegenwart von granulierten, ganz überwiegend Eisen und geringe Mengen Aluminiumoxid enthaltenden Katalysatoren und Ammoniak als Lösungsmittel zu Hexamethylendiamin zu hydrieren. In den Beispielen in Tabelle 1, Versuch 2, und Tabelle 2, Versuche 1 bis 3, werden bei Drücken von 340 atm Hexamethylendiaminausbeuten von 98,8 %, 98,8 %, 97,7 % und 97,7 % erreicht. In den ersten drei Beispielen wird vollständiger Umsatz, im vierten Beispiel 99,9%iger Umsatz erzielt. Zur Standzeit der Eisenkatalysatoren geht aus den Tabellen 1 und 2 lediglich hervor, daß die Katalysatoraktivität am Ende der Versuche (nach rund 80 bis 120 Stunden Versuchszeit) hoch ist.

Aus U.S. 4.064.172 ist weiterhin bekannt, Adipodinitril in Gegenwart von Eisenkatalysatoren, die ausgehend von Magnetit synthetisiert wurden, und Ammoniak bei Drücken von 20 bis 500 bar und Temperaturen von 80 bis 200 °C zu Hexamethylendiamin zu hydrieren. In Beispiel 1 wird eine Hexamethylendiaminausbeute von 98,2 % erreicht.

U.S. 4.282.381 beschreibt die Hydrierung von Adipodinitril zu Hexamethylendiamin mit Wasserstoff bei Temperaturen von 110 bis 220°C und einem Druck von etwa 340 atm in Gegenwart von Ammoniak und Eisenkatalysatoren. Der Hydrieraustrag enthält 0,04 bis 0,09 Gew.-% Adipodinitril und 0,2 bis 0,5 Gew.-% 6-Aminocapronitril.

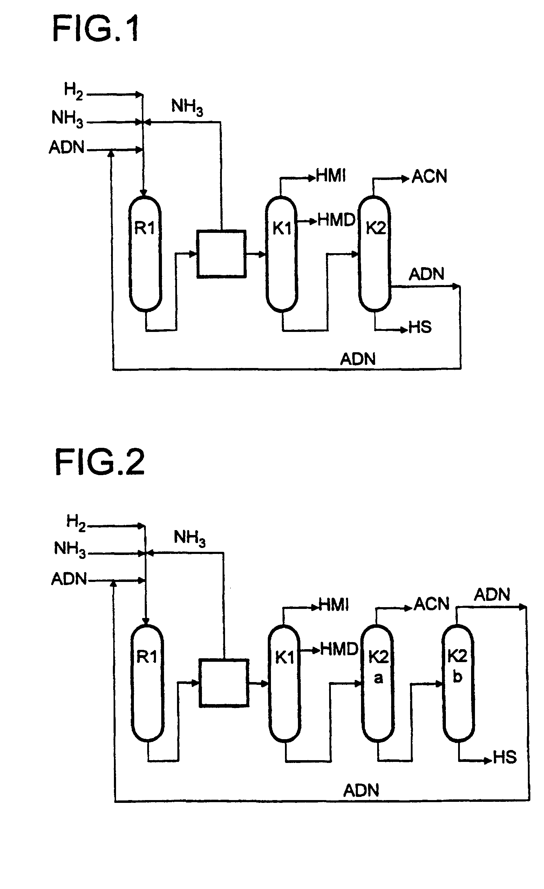

In McKetta, Encyclopedia of Chemical Processing and Design, Marcel Dekker Inc. 1987, Band 26, Seite 230, Tabelle 3, wird bestätigt, daß ein typisches Hydrierprodukt 0,01 bis 0,11 Gew.-% Adipodinitril und 0,10 bis 0,21 Gew.-% Aminocapronitril enthält. Aus den Abbildungen 2 und 4 geht hervor, daß diese kleinen Aminocapronitrilmengen abgetrennt und in die Hydrierung zurückgeführt werden können.

Aus diesen Verfahren ist bekannt, daß die Reaktionsbedingungen bei der technischen Herstellung von Hexamethylendiamin darauf gerichtet sein müssen, einen vollständigen Umsatz des Adipodinitrils und des als Zwischenprodukt der Hydrierung entstehenden 6-Aminocapronitrils zu erzielen.

Nachteilig hierbei ist, daß hierzu eine relativ hohe Temperatur und ein sehr hoher Reaktionsdruck erforderlich sind. Sinkt der Umsatz des Adipodinitrils und der des 6-Aminocapronitrils im Verlauf der Hydrierung merklich ab, so muß der Umsatz durch Steigerung der Temperatur und gegebenenfalls des Reaktionsdruckes und/oder Senkung der Katalysatorbelastung wieder angehoben werden. Andernfalls entsteht ein nicht unerheblicher Wertprodukt-Verlust.

Lassen sich die Temperatur wegen abnehmender Hexamethylendiaminselektivität und/oder der Druck aus technischen Gründen nicht weiter steigern, um vollständigen Umsatz zu erzielen, so muß die Katalysatorbelastung zurückgenommen werden. Dies bedeutet jedoch, daß die Katalysatorproduktivität, d.h. die pro Zeiteinheit produzierte Hexamethylendiamin-Menge, zurückgeht. wird eine bestimmte Produktivität unterschritten, so muß die Hydrieranlage abgestellt, der Eisenkatalysator ausgebaut und durch einen ungebrauchten oder regenerierten Katalysator ersetzt werden. Je häufiger pro Jahr derartige Abstellungen notwendig sind, desto weniger Hexamethylendiamin kann in einer bestehenden Produktionsanlage pro Jahr erzeugt werden.

Aufgabe der vorliegenden Erfindung war daher, ein Verfahren zur katalytischen Hydrierung von Adipodinitril zu Hexamethylendiamin in Gegenwart von ganz überwiegend elementares Eisen enthaltenen Katalysatoren und Ammoniak als Lösungsmittel zur Verfügung zu stellen, das die Herstellung von Hexamethylendiamin auf wirtschaftliche und technisch einfache Weise unter Vermeidung der genannten Nachteile ermöglicht.

Diese Aufgabe wird gelöst durch das eingangs definierte Verfahren.

Bei dem erfindungsgemäßen Verfahren ist es nicht notwendig, einen vollständigen Adipodinitril- und 6-Aminocapronitrilumsatz herbeizuführen. Hierdurch werden im Vergleich zum Stand der Technik deutlich höhere Katalysatorstandzeiten bei niedrigeren Drücken, geringere Abstellzeiten der Hydrieranlage und damit deutlich höhere Hexamethylendiaminproduktivitäten erzielt.

Es war nicht vorauszusehen und damit überraschend, daß die Rückführung von 6-Aminocapronitril, Adipodinitril oder deren Gemische in die Hydrierstufe keine Verschlechterung der Katalysatorstandzeit bewirkt. Außerdem war überraschend, daß durch die gesamte Rückführung keine störende Aufpegelung von Nebenprodukten erfolgt.

Das in dem erfindungsgemäßen Verfahren eingesetzte Adipodinitril kann im allgemeinen nach den an sich bekannten Verfahren, vorzugsweise durch Umsetzung von Butadien mit Blausäure in Gegenwart von Katalysatoren, insbesondere Nickel-(0)-Komplexverbindungen und phosphorhaltigen Cokatalysatoren, über Pentennitril als Zwischenstufe, hergestellt werden.

Als Katalysatoren können in an sich üblicher Weise Eisenkatalysatoren verwendet werden, die für die Herstellung von Hexamethylendiamin durch Hydrierung von Adipodinitril bekannt sind. Bevorzugte Katalysatorvorläufer sind solche, die zu 90 bis 100 Gew.-% , bevorzugt 92 bis 99 Gew.-%, bezogen auf die Gesamtmasse des Katalysatorvorläufers Eisenoxide, Eisen-(II, III)-oxid, Eisen-(II)-oxid, Eisen-(II)-hydroxid, Eisen-(III)-hydroxid oder Eisenoxyhydroxid wie FeOOH bestehen. Verwendet werden können synthetisch hergestellte oder natürlich vorkommende Eisenoxide, Eisenhydroxide oder Eisenoxyhydroxide, Magneteisenstein (Magnetit), der im Idealfall mit Fe₃O₄ beschrieben werden kann, Brauneisenstein, der im Idealfall mit Fe₂O₃ x H₂O beschrieben werden kann, oder Roteisenstein (Hämatit), der im Idealfall mit Fe₂O₃ beschrieben werden kann.

Bevorzugte Katalysatoren sind solche, die
a) Eisen oder eine Verbindung auf der Basis von Eisen oder deren Gemische enthalten und
b) von 0,001 bis 5 Gew.-% bezogen auf a) eines Promotors auf der Basis von 2, 3, 4, 5 oder 6 Elementen ausgewählt aus der Gruppe bestehend aus Aluminium, Silizium, Zirkonium, Titan, Vanadium, Mangan sowie
c) von 0 bis 5 Gew.-% bezogen auf a) einer Verbindung auf der Basis eines Alkali- oder Erdalkalimetalls, enthalten.

Bevorzugte Katalysatorvorläufer sind weiterhin solche, in denen Komponente b) von 0,001 bis 5 Gew.-%, vorzugsweise 0,01 bis 4 Gew.-%, insbesondere 0,1 bis 3 Gew.-% eines Promotors auf der Basis von 2, 3, 4, 5 oder 6 Elementen ausgewählt aus der Gruppe bestehend aus Aluminium, Zirkonium, Silizium, Titan, Mangan und Vanadium, enthält.

Bevorzugte Katalysatorvorläufer sind weiterhin solche, in denen Komponente c) von 0 bis 5 Gew.-%, vorzugsweise 0,1 bis 3 Gew.-%, eine Verbindung auf der Basis eines Alkali- oder Erdalkalimetalls, vorzugsweise ausgewählt aus der Gruppe bestehend aus Lithium, Natrium, Kalium, Rubidium, Cäsium, Magnesium und Calcium enthält.

Bei den Katalysatoren kann es sich um Voll- oder Trägerkatalysatoren handeln. Als Trägermaterialien kommen beispielsweise poröse Oxide wie Aluminiumoxid, Siliziumoxid, Alumosilikate, Lanthanoxid, Titandioxid, Zirkondioxid, Magnesiumoxid, Zinkoxid und Zeolithe sowie Aktivkohle oder Mischungen davon in Betracht.

Die Herstellung erfolgt in der Regel derart, daß man Vorläufer der Komponente a) gewünschtenfalls zusammen mit Vorläufern der Promotorenkomponenten b) und gewünschtenfalls mit Vorläufern der Spurenkomponenten c) in Gegenwart oder Abwesenheit von Trägermaterialien (je nachdem, welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten a), b) und gewünschtenfalls c) tränkt, wobei man die einzelnen Komponenten gleichzeitig oder nacheinander zugeben kann, oder indem man die Komponenten a), gewünschtenfalls b) und c) auf den Träger nach an sich bekannten Methoden aufsprüht.

Als Vorläufer der Komponenten a) kommen in der Regel gut wasserlösliche Salze des Eisens wie Nitrate, Chloride, Acetate, Formiate und Sulfate in Betracht, vorzugsweise Nitrate.

Als Vorläufer der Komponenten b) kommen in der Regel gut wasserlösliche Salze oder Komplexsalze der zuvor genannten Metalle und Halbmetalle wie Nitrate, Chloride, Acetate, Formiate und Sulfate in Betracht, vorzugsweise Nitrate.

Als Vorläufer der Komponenten c) kommen in der Regel gut wasserlösliche Salze der zuvor genannten Alkalimetalle und Erdalkalimetalle wie Hydroxyde, Carbonate, Nitrate, Chloride, Acetate, Formiate und Sulfate in Betracht, vorzugsweise Hydroxyde und Carbonate.

Die Fällung erfolgt im allgemeinen aus wäßrigen Lösungen, wahlweise durch Zugabe von Fällungsreagenzien, durch Änderung des pH-Werts oder durch Änderung der Temperatur.

Üblicherweise trocknet man die so erhaltene Katalysatorvormasse im allgemeinen bei Temperaturen im Bereich von 80 bis 150°C, vorzugsweise 80 bis 120°C.

Das Calcinieren nimmt man üblicherweise bei Temperaturen im Bereich von 150 bis 500°C, vorzugsweise von 200 bis 450°C in einem Gasstrom aus Luft oder Stickstoff vor.

Nach dem Calcinieren setzt man die erhaltene Katalysatormasse im allgemeinen einer reduzierenden Atmosphäre aus ("Aktivierung"), beispielsweise indem man sie bei einer Temperatur im Bereich von 200 bis 500°C, vorzugsweise 250 bis 400°C 2 bis 100 Stunden einer Wasserstoffatmosphäre oder einer Gasmischung, enthaltend Wasserstoff und ein Inertgas wie Stickstoff, aussetzt. Die Katalysatorbelastung beträgt hierbei bevorzugt 200 l pro Liter Katalysator.

Die Aktivierung von Eisenkatalysatoren durch Reduktion von Eisenoxiden mit Wasserstoff kann in an sich bekannter Weise, beispielsweise gemäß U.S. 3,758,584, bei 300 bis 600°C mit Gemischen aus Wasserstoff und Ammoniak, durchführt werden oder gemäß U.S. 4,480,051 dreistufig erfolgen, indem man in einem ersten Schritt das Eisenoxid mit Wasserstoff oder Gemischen aus Wasserstoff und Ammoniak reduziert, dann das gebildete elementare Eisen in einem zweiten Schritt mit einem Sauerstoff enthaltenden Gas behandelt und anschließend in einem dritten Schritt die Reduktion des ersten Schrittes wiederholt.

Eine weitergehende Stabilisierung von reduzierten pyrophoren Eisenkatalysatoren, z. B. um sie zu transportieren, ist in U.S. 3.986.985 beschrieben. Durch eine kurzzeitige Behandlung des stabilisierten Eisenkatalysators mit Wasserstoff kann die ursprüngliche Katalysatoraktivität wiederhergestellt werden.

Vorteilhaft führt man die Aktivierung des Katalysators direkt im Synthesereaktor durch, da hierdurch üblicherweise der ansonsten erforderliche Zwischenschritt, nämlich die Passivierung der Oberfläche bei üblicherweise Temperaturen im Bereich von 20 bis 80°C, vorzugsweise von 25 bis 35°C mittels Stickstoff-Sauerstoff-Mischungen wie Luft, wegfällt. Die Aktivierung passivierter Katalysatoren nimmt man dann bevorzugt im Synthesereaktor bei einer Temperatur im Bereich von 180 bis 500°C, vorzugsweise von 200 bis 400°C in einer wasserstoffhaltigen Atmosphäre vor.

Die Katalysatoren können bevorzugt als Festbettkatalysatoren in Sumpf- oder Rieselfahrweise oder auch als Suspensionskatalysatoren eingesetzt werden.

Die Hydrierung kann diskontinuierlich, bevorzugt aber kontinuierlich mit suspendierten, bevorzugt aber fest angeordneten Katalysatoren in Gegenwart von Ammoniak durchgeführt werden.

Arbeitet man mit fest angeordneten Katalysatoren, so kann der Festbettreaktor R 1 (siehe Abbildungen 1 und 2) in Riesel- oder Sumpffahrweise betrieben werden. Dabei ist die Fahrweise im geraden Durchgang durch einen Reaktor oder mehrere hintereinander geschaltete Reaktoren mit oder ohne Zwischenkühlung oder eine Fahrweise mit einem Reaktor oder mehreren Reaktoren mit Produktrückführung in einem Flüssigkeitskreislauf um den oder die Reaktoren möglich.

Die Reaktionstemperatur beträgt im allgemeinen 70 bis 220°C, insbesondere 80 bis 170 °C, und der Druck 100 bis 400 bar, insbesondere 150 bis 350 bar, besonders bevorzugt 200 bis 250 bar.

Die Katalysatorbelastung beträgt üblicherweise 0,1 bis 3 kg Adipodinitril/l Kat. x h, insbesondere 0,5 bis 2 kg Adipodinitril/l Kat. x h.

Die Parameter, wie Temperatur, Druck und Katalysatorbelastung, zur Einstellung der erfindungsgemäßen Summe der 6-Aminocapronitril- und Adipodinitril-Konzentration, bezogen auf das ammoniakfreie Hydriergemisch, im Reaktoraustrag von 1 bis 50 Gew.-%, vorzugsweise 2 bis 40 Gew.-%, besonders bevorzugt 3 bis 40 Gew.-%, insbesondere 5 bis 30 Gew.-% können durch wenige einfache Vorversuche leicht ermittelt werden.

Aus dem Hydrieraustrag aus Schritt a) wird in Schritt b) der Ammoniak in üblicher Weise, vorzugsweise destillativ, abgetrennt, wie beispielsweise in DE19 548289 beschrieben. Der Ammoniak kann anschließend vorteilhaft in Schritt a) wieder eingesetzt werden.

Anschließend trennt man in üblicher Weise, vorzugsweise destillativ, aus dem Gemisch Hexamethylendiamin und das Nebenprodukt Hexamethylenimin ab. Im Falle einer destillativen Abtrennung kann diese in mehreren , wie zwei oder drei, Kolonnen oder vorzugsweise einer Kolonne (K1) erfolgen.

Das in Schritt c) erhaltene Hexamethylendiamin kann anschließend in an sich bekannter Weise, vorzugsweise destillativ, gereinigt werden.

Der nach Schritt c) verbleibende Produktstrom enthält Adipodinitril, 6-Aminocapronitril, Nebenprodukte und Verbindungen, die einen Siedepunkt über dem von Adipodinitril aufweisen ("Hochsieder"). Hierzu gehören Stickstoffbasen, wie 2-(5-Cyanopentylamino)-tetrahydroazepin und 2-(6-Aminohexylamino)-tetrahydroazepin. Aus diesem Produktstrom trennt man gemäß Schritt d) in üblicher Weise, vorzugsweise destillativ, 6-Aminocapronitril und Adipodinitril einzeln oder gemeinsam von Hochsiedern ab. Im Falle einer destillativen Abtrennung kann diese in mehreren , wie zwei (K2a und K2b in Abbildung 2) oder drei, Kolonnen oder einer Kolonne (K2 in Abbildung 1) erfolgen. Im Falle von einer Kolonne (K2) kann vorteilhaft Adipodinitril über einen Seitenabzug, 6-Aminocapronitril über Kopf und Hochsieder als Sumpf erhalten werden.
Aus Adipodinitril können in Gegenwart der in den Sumpfprodukten enthaltenen Stickstoffbasen, , wie 2-(5-Cyanopentylamino)-tetrahydroazepin und 2-(6-Aminohexylamino)-tetrahydroazepin, deutliche Mengen an 1-Amino-2-cyano-cyclopenten entstehen. Aus reinem Adipodinitril entstehen dagegen bei Sumpftemperaturen von 200°C nur geringe Mengen an 1-Amino-2-cyano-cyclopenten.

Der Gehalt an 1-Amino-2-cyano-cyclopenten, bezogen auf Adipodinitril, in dem in Schritt a) eingesetzten Adipodinitril, welches frisches Adipodinitril und aus gemäß Schritt e) zurückgeführtes 6-Aminocapronitril, Adipodinitril oder deren Gemische enthält, sollte unter 5000 Gew.-ppm, vorteilhaft zwischen 10 und 5000 Gew.-ppm, bevorzugt zwischen 10 und 3000 Gew.-ppm, besonders bevorzugt zischen 10 und 1500 Gew.-ppm, insbesondere zwischen 10 und 100 Gew.-ppm liegen.

Durch Absenkung des Gehalts an 1-Amino-2-cyano-cyclopenten in dem in Schritt a) eingesetzten Adipodinitril, welches frisches Adipodinitril und aus gemäß Schritt e) zurückgeführtes 6-Aminocapronitril, Adipodinitril oder deren Gemische enthält, wird die Ausbeute an 6-Aminocapronitril und Hexamethylendiamin gesteigert und die Reinigung von Hexamethylendiamin erleichtert.

Im Falle einer destillativen Abtrennung sollte die Sumpftemperatur vorteilhaft unter 220°C, vorzugsweise unter 190°C, insbesondere unter 185°C liegen, wobei sich wegen des geringen Dampfdrucks der zu trennenden Verbindungen eine Sumpftemperatur von mindestens 100°C, vorzugsweise mindestens 140°C, insbesondere mindestens 160°C empfiehlt. Die Drücke im Sumpf der Kolonne sollten vorteilhaft 0,1 bis 100, insbesondere 5 bis 40 mbar betragen. Vorzugsweise sollten die Verweilzeiten der Sumpfprodukte in der Destillation 1 bis 60, insbesondere 5 bis 15 Minuten betragen.

In einer bevorzugten Ausführungsform werden diese Destillationsbedingungen auf die Abtrennung des Adipodinitrils von Hochsiedern angewendet.

In einer bevorzugten Ausführungsform enthält das Sumpfprodukt 1 bis 80 Gew.-% Adipodinitril, bezogen auf Hochsieder. Aus diesem Produktstrom kann anschließend vorteilhaft Adipodinitril in einem Verdampfer bei einem Druck von 1 bis 50 mbar, vorzugsweise 2 bis 25 mbar, weiteres Adipodinitril erhalten werden.

Gemäß Schritt e) werden 6-Aminocapronitril, Adipodinitril oder deren Gemische in den Schritt a) zurückgeführt.

Im Sinne der vorliegenden Erfindung kann ebenfalls aus dem nach Schritt b) erhaltenen Gemisch Hexamethylendiamin zusammen mit 6-Aminocapronitril abgetrennt und anschließend die Mischung in die beiden Komponenten aufgetrennt werden.

In einer weiteren bevorzugten Ausführungsform entfernt man aus dem in den Schritt a) zurückzuführenden Adipodinitril enthaltenden Strom in an sich bekannter Weise, beispielsweise destillativ oder extraktiv Nebenprodukte, insbesondere 1-Amino-2-cyano-cyclopenten.

In einer weiteren bevorzugten Ausführungsform reinigt man den in den Schritt a) zurückzuführenden Adipodinitril enthaltenden Strom in an sich bekannter Weise, beispielsweise durch Behandlung mit einer anorganischen Säure, wie Mineralsäure, organischen Säure, wie Carbonsäure, oder einem sauren Ionentauscher oder durch Behandlung mit einem Oxidationsmittel, wie Luft, Ozon, Wasserstoffperoxid oder einem anorganischen oder organischen Peroxid.

Überraschenderweise wurde bei dem erfindungsgemäßen Verfahren festgestellt, daß das Verfahren zu deutlichen Vorteilen bei der Hydrierung, der destillativen Reinigung von Hexamethylendiamin und der Standzeit des Hydrierkatalysators führt.

## Patentansprüche

1. Verfahren zur katalytischen Hydrierung von Adipodinitril zu Hexamethylendiamin bei erhöhter Temperatur und erhöhtem Druck in Gegenwart von Katalysatoren, die auf elementarem Eisen als katalytisch aktive Komponente basieren, und Ammoniak als Lösungsmittel, **dadurch gekennzeichnet, daß** man
a) Adipodinitril bei Temperaturen von 70 bis 220°C und Drücken von 100 bis 400 bar in Gegenwart von Katalysatoren, die auf elementarem Eisen als katalytisch aktive Komponente basieren, und Ammoniak als Lösungsmittel zu einer Mischung enthaltend Adipodinitril, 6-Amincapronitril, Hexamethylendiamin und Hochsieder hydriert, bis die Summe aus der 6-Aminocapronitril- und der Adipodinitril-Konzentration von 1 bis 50 Gew.-%, bezogen auf das ammoniakfreie Hydriergemisch, beträgt,
b) aus dem Hydrieraustrag Ammoniak abtrennt,
c) aus dem verbleibenden Gemisch Hexamethylendiamin abtrennt,
d) 6-Aminocapronitril und Adipodinitril von Hochsiedern einzeln oder gemeinsam abtrennt und
e) 6-Aminocapronitril, Adipodinitril oder deren Gemische in den Schritt a) zurückführt.

2. Verfahren nach Anspruch 1, wobei man die Abtrennung des Adipodinitrils von Hochsiedern destillativ bei Sumpftemperaturen von unter 220°C durchführt.

3. Verfahren nach Anspruch 2, wobei man die Abtrennung des Adipodinitrils von Hochsiedern destillativ bei Sumpftemperaturen von unter 185°C durchführt.

4. Verfahren nach den Ansprüchen 1 bis 3, wobei man die Abtrennung des Adipodinitrils von Hochsiedern destillativ durchführt und in dem als Sumpfprodukt erhaltenen Hochsiederstrom einen Gehalt an Adipodinitril von 1 bis 80 Gew.-%, bezogen auf den Hochsiedergehalt, einstellt.

5. Verfahren nach Anspruch 4, wobei man aus dem Adipodinitril enthaltenden Hochsiederstrom in einem nachfolgenden Verdampfer bei einem Druck von 1 bis 50 mbar den Hauptanteil des darin enthaltenen Adipodinitrils abtrennt.

6. Verfahren nach den Ansprüchen 1 bis 5, wobei man zusätzlich zwischen den Schritten d) und e) den Gehalt des als Nebenprodukt enthaltenen 1-Amino-2-cyano-cyclopenten in dem Adipodinitril enthaltenden Strom reduziert.

7. Verfahren nach den Ansprüchen 1 bis 6, wobei man zusätzlich zwischen den Schritten d) und e) den Adipodinitril enthaltenden Strom mit einer Säure behandelt.

8. Verfahren nach den Anspruch 7, wobei man als Säure eine Mineralsäure, eine Carbonsäure oder einen sauren Ionentauscher verwendet.

9. Verfahren nach den Ansprüchen 1 bis 8, wobei man zusätzlich zwischen den Schritten d) und e) den Adipodinitril enthaltenden Strom mit einem Oxidationsmittel behandelt.

10. Verfahren nach Anspruch 9, wobei man als Oxidationsmittel Luft, Ozon, Wasserstoffperoxid oder ein anorganisches oder organisches Peroxid verwendet.

11. Verfahren nach den Ansprüchen 1 bis 10, wobei der Gehalt in dem in Schritt a) eingesetzten Adipodinitril, welches frisches Adipodinitril und aus gemäß Schritt e) zurückgeführtes 6-Aminocapronitril, Adipodinitril oder deren Gemische enthält, an 1-Amino-2-cyano-cyclopenten unter 5000 Gew.-ppm bezogen auf Adipodinitril liegt.

## Claims

1. A process for catalytic hydrogenation of adiponitrile to hexamethylenediamine at elevated temperature and elevated pressure in the presence of catalysts based on elemental iron as catalytically active component and ammonia as solvent, which comprises
a) hydrogenating adiponitrile at from 70 to 220°C and from 100 to 400 bar in the presence of catalysts based on elemental iron as catalytically active component and ammonia as solvent to obtain a mixture comprising adiponitrile, 6-aminocapronitrile, hexamethylenediamine and high boilers until the sum total of the 6-aminocapronitrile concentration and the adiponitrile concentration is within the range from 1 to 50% by weight, based on the ammonia-free hydrogenation mixture,
b) removing ammonia from the hydrogenation effluent,
c) removing hexamethylenediamine from the remaining mixture,
d) separating 6-aminocapronitrile and adiponitrile from high boilers individually or together, and
e) returning 6-aminocapronitrile, adiponitrile or mixtures thereof into step a).

2. A process as claimed in claim 1, wherein the separating of the adiponitrile from high boilers is effected distillatively at base of column temperatures of below 220°C.

3. A process as claimed in claim 2, wherein the separating of the adiponitrile from high boilers is effected distillatively at base of column temperatures of below 185°C.

4. A process as claimed in any of claims 1 to 3, wherein the separating of the adiponitrile from high boilers is effected distillatively and the high boilers stream obtained as bottom product is set to an adiponitrile content of from 1 to 80% by weight, based on the high boiler content.

5. A process as claimed in claim 4, wherein the main fraction of the adiponitrile in the stream of high boilers and adiponitrile is removed from the stream in a downstream evaporator at from 1 to 50 mbar.

6. A process as claimed in any of claims 1 to 5, further comprising reducing the level of 1-amino-2-cyanocyclopentene by-product in the adiponitrile stream between steps d) and e).

7. A process as claimed in any of claims 1 to 6, further comprising treating the adiponitrile stream with an acid between steps d) and e).

8. A process as claimed in claim 7, wherein the acid used is a mineral acid, a carboxylic acid or an acidic ion exchanger.

9. A process as claimed in any of claims 1 to 8, further comprising treating the adiponitrile stream with an oxidizing agent between steps d) and e).

10. A process as claimed in claim 9, wherein the oxidizing agent used is air, ozone, hydrogen peroxide or an inorganic or organic peroxide.

11. A process as claimed in any of claims 1 to 10, wherein the 1-amino-2-cyanocyclopentene content of the adiponitrile used in step a), which comprises fresh adiponitrile and 6-aminocapronitrile, adiponitrile or mixtures thereof returned from step e), is below 5000 weight ppm based on adiponitrile.

## Revendications

1. Procédé d'hydrogénation catalytique d'adipodinitrile en hexaméthylènediamine à température élevée et pression élevée, en présence de catalyseurs qui sont basés sur du fer élémentaire comme composant catalytiquement actif, et d'ammoniac comme solvant, **caractérisé en ce que**
a) on hydrogène de l'adipodinitrile à des températures de 70 à 220°C et à des pressions de 100 à 400 bars en présence de catalyseurs, qui sont basés sur du fer élémentaire comme composant catalytiquement actif, et d'ammoniac comme solvant pour former un mélange contenant de l'adipodinitrile, du 6-aminocapronitrile, de l'hexaméthylène-diamine et des substances à point d'ébullition élevé, jusqu'à ce que la somme de la concentration de 6-aminocapronitrile et de la concentration d'adiponitrile soit de 1 à 50% en poids, par rapport au mélange d'hydrogénation exempt d'ammoniac,
b) on isole de l'ammoniac du produit de sortie de l'hydrogénation,
c) on isole de l'hexaméthylènediamine du mélange subsistant,
d) on isole, séparément ou conjointement, du 6-aminocapronitrile et de l'adipodinitrile des substances à point d'ébullition élevé, et
e) on recycle du 6-aminocapronitrile, de l'adipodinitrile ou leurs mélanges dans l'étape a).

2. Procédé suivant la revendication 1, dans lequel on effectue l'isolement de l'adipodinitrile à partir des substances à point d'ébullition élevé par distillation à des températures de fond de cuve inférieures à 220°C.

3. Procédé suivant la revendication 2, dans lequel on effectue l'isolement de l'adipodinitrile à partir des substances à point d'ébullition élevé par distillation à des températures de fond de cuve inférieures à 185°C.

4. Procédé suivant l'une des revendications 1 à 3, dans lequel on effectue l'isolement de l'adipodinitrile à partir des substances à point d'ébullition élevé par distillation et on ajuste, dans le courant de substances à point d'ébullition élevé obtenu comme produit de fond de cuve, une teneur en adipodinitrile de 1 à 80% en poids, par rapport à la teneur en substances à point d'ébullition élevé.

5. Procédé suivant la revendication 4, dans lequel, à partir du courant de substances à point d'ébullition élevé contenant de l'adipodinitrile, on isole, dans un évaporateur consécutif à une pression de 1 à 50 mbars, la fraction principale de l'adipodinitrile contenu dedans.

6. Procédé suivant l'une des revendications 1 à 5, dans lequel, entre les étapes d) et e), on réduit en supplément la teneur en le 1-amino-2-cyano-cyclopentène contenu comme produit secondaire dans le courant contenant de l'adipodinitrile.

7. Procédé suivant l'une des revendications 1 à 6, dans lequel, entre les étapes d) et e), on traite en supplément le courant contenant de l'adipodinitrile par un acide.

8. Procédé suivant la revendication 7, dans lequel on utilise, comme acide, un acide minéral, un acide carboxylique ou un échangeur d'ions acides.

9. Procédé suivant l'une des revendications 1 à 8, dans lequel, entre les étapes d) et e), on traite en supplément le courant contenant de l'adipodinitrile par un agent oxydant.

10. Procédé suivant la revendication 9, dans lequel on utilise, comme agent oxydant, de l'air, de l'ozone, du peroxyde d'hydrogène ou un peroxyde inorganique ou organique.

11. Procédé suivant l'une des revendications 1 à 10, dans lequel la teneur en 1-amino-2-cyano-cyclopentène dans l'adipodinitrile mis en oeuvre dans l'étape a), qui contient de l'adipodinitrile frais et du 6-aminocapronitrile, de l'adipodinitrile ou leurs mélanges recyclés conformément à l'étape e), est située en dessous de 5.000 ppm en poids par rapport à l'adipodinitrile.
